# EUROPEAN PATENT APPLICATION

(11) **EP 4 732 761 A1**
(43) Date of publication of application: **29.04.2026**
(21) Application number: 25205366.5
(22) Date of filing: 29.09.2025
(51) Int. Cl.: A61B 5/00

(54) **VENTLESS TOCODYNAMOMETER**

(30) Priority: 24.10.2024 US 202418925310
(71) Applicant: GE Precision Healthcare LLC, Waukesha, WI 53188 (US)
(72) Inventor: DHULIPALLA, Ravi Kumar, Waukesha, 53188 (US); M S, Sivakumar, Waukesha, 53188 (US); JONNADULA, Bharath Kumar, Waukesha, 53188 (US); KAMATH, Harish, Waukesha, 53188 (US)
(74) Representative: Kilburn & Strode LLP

(57) **Abstract**

A tocodynamometer (toco) has a housing configured to be maintained against the abdominal skin of the maternal patient, wherein the housing forms a sealed cavity. A sensing membrane is on a front side of the housing, wherein the sensing membrane is configured to be in contact with the abdominal skin and to deform inward during uterine contractions of the maternal patient. A sensor is located in the sealed cavity of the housing and configured to provide an output based on the inward deformation of the sensing membrane. A processor is within the sealed cavity and configured to receive the output of the sensor and to generate a uterine activity value based thereon. A pressure diffusing membrane is on at least one side of the housing and is configured to expand outward when the sensing membrane deforms inward to mitigate pressure increase within the sealed cavity.

## Description

### BACKGROUND

The present disclosure generally relates to tocodynamometers, and specifically to a tocodynamometer with a sealed cavity having no opening configured to vent pressure.

The tocodynamometer (toco) is a device configured to be worn against the abdomen of a maternal patient and is configured to measure uterine contractions by measuring the displacement of the external surface of the abdomen. A toco configured for measuring uterine contractions on a maternal patient during labor typically involves a movable patient contacting member that is placed against the skin of the maternal patient's abdomen and a transducer, such as a strain gauge, for converting displacement of the contacting member into an electrical output.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one aspect of the disclosure, a tocodynamometer (toco) is configured to measure uterine activity of a maternal patient and has a housing configured to be maintained against abdominal skin of the maternal patient, wherein the housing forms a sealed cavity and comprises a front side configured to contact the abdominal skin, a back side opposite the front side, and a lateral side connecting the front side to the back side. A sensing membrane is on the front side of the housing, wherein the sensing membrane is configured to be in contact with the abdominal skin and to deform inward during uterine contractions of the maternal patient. A sensor is located in the sealed cavity of the housing and configured to provide an output based on the inward deformation of the sensing membrane. A processor is within the sealed cavity and configured to receive the output of the sensor and to generate a uterine activity value based thereon. A pressure diffusing membrane is on at least one side of the housing and is configured to expand outward when the sensing membrane deforms inward to mitigate pressure increase within the sealed cavity due to the inward deformation of the sensing membrane.

In one embodiment, the sensor includes a linear variable differential transformer (LVDT) configured to sense the inward deformation of the sensing membrane and a spring functionally connected to the sensor and configured to absorb the inward deformation of the sensing membrane, wherein the spring is configured to minimize the inward deformation of the sensing membrane.

In another embodiment, the spring is configured to minimize the inward deformation of the sensing membrane in response to a maximum expected force from the contractions of the maternal patient. Optionally, the spring is configured to minimize the inward deformation of the sensing membrane in response to a maximum expected force from the contractions of the maternal patient to less than 1 mm.

In another embodiment, a spring constant of a spring in a spring loaded LVDT sensor configured to sense the inward deformation of the sensing membrane is 200 lbs/in.

In another embodiment, the sensor is a piezoelectric sensor which is configured to minimize the inward deformation of the sensing membrane.

In another embodiment, at least two pressure accommodation membranes, each on a different portion of the housing.

In another embodiment, the housing is cylindrical with the front side being flat and the lateral side forming a ring around the front side, wherein the two pressure accommodation membranes are on opposite portions of the lateral side of the housing.

In another embodiment, the at least one pressure diffusing membrane is configured to expand outward to add a total volume within the sealed cavity that is equal to a volume displaced by the inward deformation of the sensing membrane.

In another embodiment, a total area of the at least two pressure accommodation membranes is greater than or equal to an area of the sensing membrane.

In another embodiment, the pressure accommodation membrane is comprised of the same material as the sensing membrane.

In another embodiment, the sealed cavity is watertight.

In another embodiment, the sealed cavity further comprising a battery and a wireless transmitter, wherein the battery is configured to power the processor and the wireless transmitter to wirelessly transmit the uterine activity value to a patient monitor.

In another aspect of the disclosure, a patient monitoring system is configured to monitor uterine activity of a maternal patient and includes a tocodynamometer (toco) and a patient monitor, which optionally is a wireless toco. The toco includes a housing configured to be maintained against abdominal skin of the maternal patient, wherein the housing forms a sealed cavity and comprises a front side configured to contact the abdominal skin, a back side opposite the front side, and a lateral side connecting the front side to the back side. A sensing membrane is on the front side of the housing, wherein the sensing membrane is configured to be in contact with the abdominal skin and to deform inward during uterine contractions of the maternal patient. A pressure diffusing membrane on at least one side of the housing is configured to expand outward when the sensing membrane deforms inward to mitigate pressure increase within the sealed cavity due to the inward deformation of the sensing membrane. A sensor is located in the sealed cavity of the housing and configured to provide an output based on the inward deformation of the sensing membrane. A processor within the sealed cavity is configured to receive the output of the sensor and to generate at least one uterine activity value based thereon. Where the toco is a wireless toco, a wireless transmitter is positioned in the sealed cavity and configured to wirelessly transmit the at least one uterine activity value. The patient monitor is configured to receive the at least one uterine activity value and to store and display at least one of the at least one uterine activity value.

In one embodiment, the sensor includes a linear variable differential transformer configured to sense the inward deformation of the sensing membrane and a spring functionally connected to the sensor and configured to absorb the inward deformation of the sensing membrane, wherein the spring is configured to minimize the inward deformation of the sensing membrane.

In another embodiment, the spring is configured to minimize the inward deformation of the sensing membrane in response to a maximum expected force from the contractions of the maternal patient. Optionally, the spring is configured to minimize the inward deformation of the sensing membrane to about 0.2 mm or less.

In another embodiment, the sensor is a piezoelectric sensor.

In another embodiment, at least two pressure accommodation membranes are each located on a different portion of the housing.

In another embodiment, the housing is cylindrical with the front side being flat and the lateral side forming a ring around the front side, wherein the two pressure accommodation membranes are on opposite portions of the lateral side of the housing.

In another embodiment, a total area of the at least two pressure accommodation membranes is greater than or equal to an area of the sensing membrane.

In another embodiment, the at least two pressure diffusing membranes are configured to expand outward to add a total volume within the sealed cavity that is equal to a volume displaced by the inward deformation of the sensing membrane.

In another embodiment, the at least one pressure diffusing membrane is configured to expand outward to add a total volume within the sealed cavity that is equal to a volume displaced by the inward deformation of the sensing membrane.

In another embodiment, the at least one pressure accommodation membrane is comprised of the same material as the sensing membrane.

Various other features, objects, and advantages of the invention will be made apparent from the following description taken together with the drawings.

### BRIEF DESCRIPTION OF THE DRAWINGS

The present disclosure is described with reference to the following Figures.
FIG. 1 depicts an exemplary monitoring system including a tocodynamometer (toco) positioned on a maternal patient's abdomen according to one embodiment of the present disclosure.
FIG. 2 is a cross-section of an exemplary toco with a sealed cavity.
FIG. 3 is a cross-section of an exemplary toco with a sealed cavity and having two pressure diffusing membranes according to one embodiment of the present disclosure.
FIGS. 4A-4B show another exemplary toco having a pressure diffusing membrane in accordance with another embodiment of the present disclosure.
FIG.5 depicts a maternal patient monitoring system comprising an exemplary wireless toco according to another embodiment of the present disclosure.
FIGS. 6A-6B illustrate embodiments of a toco comprising a spring-loaded linear variable differential transformer.
FIG.7 illustrates another embodiment of a toco comprising a piezoelectric sensor.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

The current inventors have endeavored to develop a wireless tocodynamometer (toco). The inventors have recognized that it is preferable for a maternal patient to be ambulatory leading up to and/or during labor, and that having wired sensors attached between the maternal patient and a patient monitor significantly inhibits movement of the maternal patient. The inventors have further recognized that development of a wireless toco requires eliminating existing venting methods. When uterine pressure is applied to the membrane, the membrane deforms inwards thereby increasing the air pressure inside the cavity of the toco. If not vented, the pressure will create an opposing force to the uterine pressure and cause inaccuracy in the measurements. To remove this opposing force, a vent hole is needed to relieve the air pressure. However, due to frequent exposure to fluids, it is preferable for the toco to have a housing providing a sealed cavity that prevents water and other fluid ingress. In existing toco devices, a long vent tube is connected from the housing to a distance away from the toco. The vent tube enables the air to escape through this vent tube when a force is applied on the sensing membrane. Typically, the vent tube is embedded in and runs along the cable connecting to the patient monitor.

The disclosed toco and patient monitoring system were developed in view of the foregoing problems and challenges recognized by the inventors. In wireless designs, the cable is eliminated and having a long vent tube is no longer practical. The disclosed toco, which may be implemented as a wireless toco, comprises a sealed cavity and provides accurate measurement of uterine activity without a traditional vent. The disclosed toco comprises at least one pressure diffusing membrane configured to expand outward when the sensing membrane deforms inward to mitigate the pressure increase within the sealed cavity due to the inward deformation of the sensing membrane. In one embodiment, the toco may comprise at least two pressure diffusing membranes, such as located on opposing portions of the lateral sides of the toco housing. The pressure diffusing membranes are configured to expand outward in opposite directions from one another when the sensing membrane deforms inward such that each pressure diffusing membrane mitigates a portion of the change in the internal area inside the housing caused by the inward movement of the sensing membrane.

Alternatively or additionally, the disclosed toco is configured to minimize the inward deformation of the sensing membrane and thus to minimize the amount of pressure change that the pressure diffusing membrane(s) must accommodate. In one embodiment, the sensing apparatus for sensing the movement of the sensing membrane is a spring-loaded linear variable differential transformer (LVDT) wherein the spring constant of the spring is increased significantly compared to prior art implementations of LVDT sensors in tocos. This increase in the spring constant reduces the travel of the sensing membrane in response to the same external force from a maternal contraction. In one embodiment, the spring constant is increased by ten times, thus reducing the travel of the sensing membrane in response to a peak force by ten times. Thus, where the sensing membrane of the prior art toco deforms inward by 2 mm in response to a maximum expected load from the contraction of the maternal patient, the disclosed toco would deform inward by only 0.2 mm in response to the same load.

In another embodiment of the disclosed toco configured to minimize the inward deformation of the sensing membrane, the sensor is a piezoelectric sensor. The piezoelectric sensor is a temperature compensated piezo sensor configured to measure the external force applied on the sensing membrane, and thus to measure the maternal contractions, with minimal travel of the sensing membrane. The piezo electric sensor is in the sealed housing and, since it can produce accurate measurements with little movement of the sensing membrane, the displaced volume of the sensing membrane is very small such that little or no compensation is needed via pressure diffusing membranes.

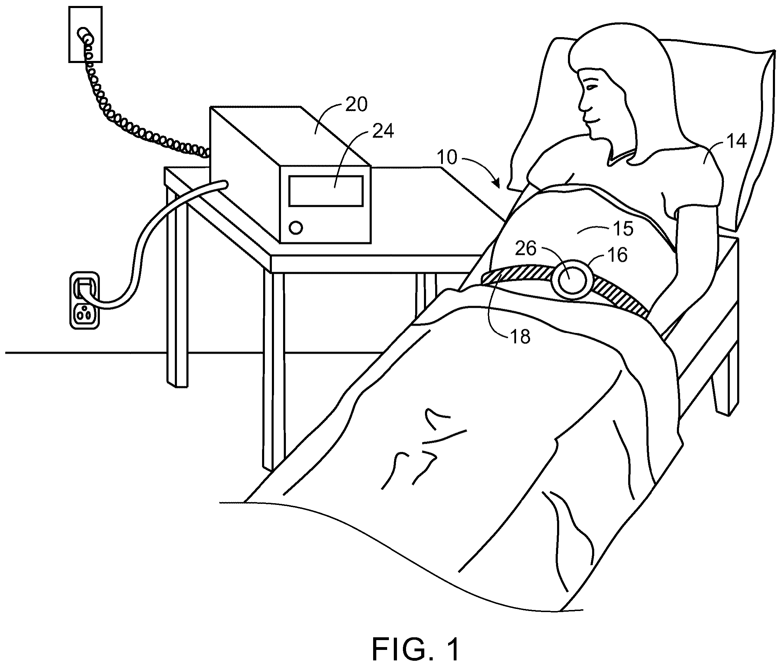

FIG. 1 depicts an exemplary embodiment of a patient monitoring system 10 for the detection of uterine activity of a maternal patient 14. In some embodiments, other physiological parameters of the maternal patient and/or the fetus may be monitored in addition to the uterine activity. The system 10 includes a tocodynamometer (toco) 16, which here is a wireless patient monitoring device configured to measure the uterine activity of the maternal patient and to wirelessly transmit the uterine activity value(s) to a patient monitor 20. In other embodiments, the disclosed ventless toco 16, which has a sealed cavity, may be used in conjunction with a wired patient monitoring system wherein the toco 16 is connected via a cable through which power is provided and/or through which data output is provided to the patient monitor or an intermediary device.

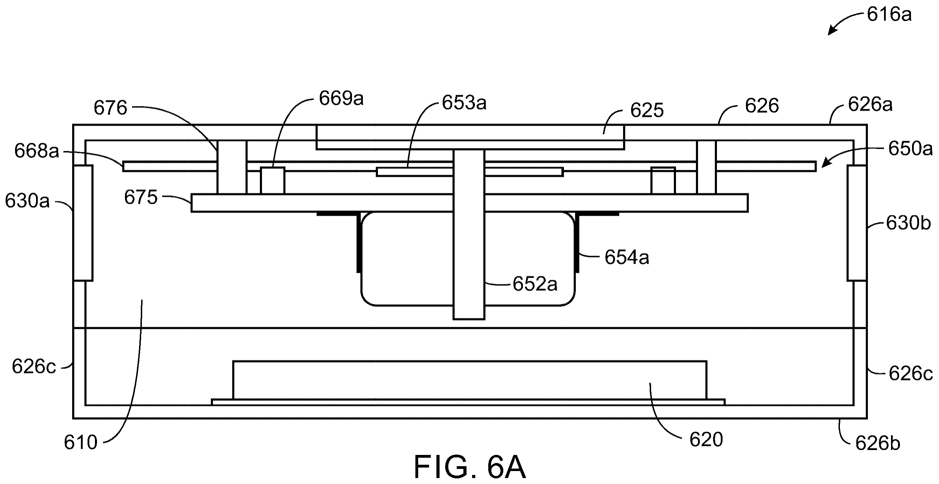

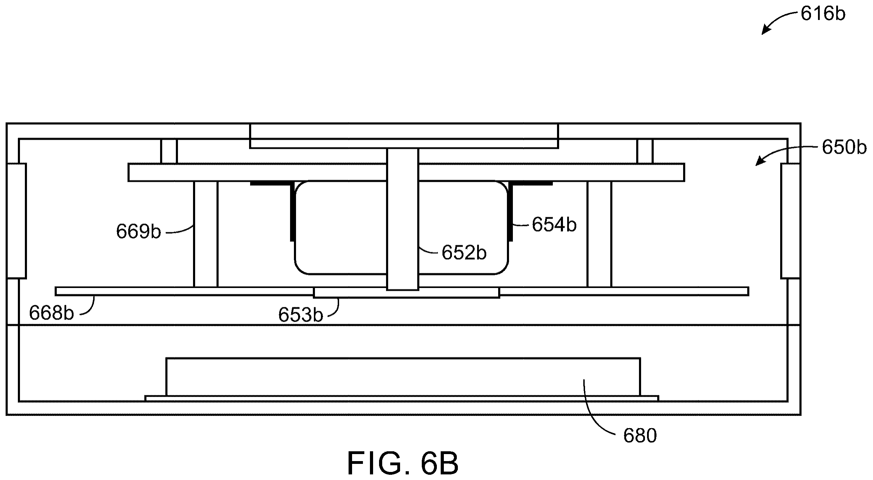

The toco 16 has a housing 26 that comprises a sensing apparatus configured to be adhered to the maternal patient's abdomen 15 to acquire maternal contraction measurements. As illustrated in FIG. 6, the toco 16 comprises a housing 26 with a sensing membrane 225 on one side thereof. The housing 26 is a rigid housing, such as made of a rigid biocompatible plastic, that defines an inner cavity 210. In the depicted embodiment, the housing is generally puck-shaped, being a short cylinder with a flat front side 226a and a flat back side 226b opposite the front side 226a. A lateral side 226c encircles the side of the housing forming a ring around the front side 226a and the back side 226b.

The toco 16 is configured such that the front side 226a is configured to be pressed against the maternal abdomen such that the sensing membrane 225 is in contact with the abdominal skin of the maternal patient. The contact is sufficient such that the sensing membrane 225 deforms inward during uterine contractions of the maternal patient. The sensing membrane 225 has a diameter dₛ, which is less than the diameter of the housing dₕ. In one embodiment, the sensing membrane 225 is centered on the front side 226a of the housing 26 and the front surface of the sensing membrane 225 is configured to contact the maternal abdomen, and thus at least a portion of the sensing membrane 225 is configured to be flush with the front side 226a of the housing when it is pressed against the maternal abdomen.

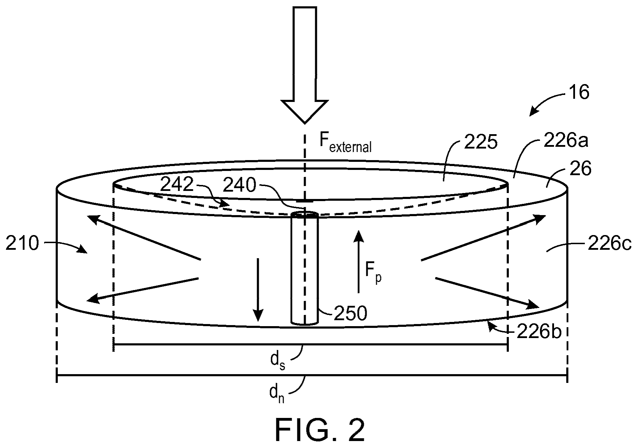

FIG. 2 illustrates the sensing membrane 225 deforming inward by an amount 240 in response to an external force Fₑₓₜₑᵣₙₐₗ. The sensing membrane 225 may be configured such that the maximum amount 240 of deformation is at the center of the sensing membrane 225. Where the external force Fₑₓₜₑᵣₙₐₗ is at a maximum expected force from the contractions of the maternal patient, then the inward deformation 240 is a maximum expected inward deformation amount. The inward deformation displaces a volume 242 within the internal cavity 210 of the housing 226, where the volume 242 increases as the amount 240 of deformation increases. To provide just one example, where the area of the sensing membrane is 480.45 mm², the maximum expected inward deformation amount 240 may be about 2 mm and the displaced volume of air is 960.91 mm³.

The housing 26 is a sealed housing configured to prevent fluid ingress and thus is ventless, meaning that the housing 26 does not vent air in or out in response to movement of the sensing membrane. Thus, without any other form of pressure mitigation, the sealed cavity inside the housing 26 will see an increase in pressure Fₚ in response to inward deformation of the sensing membrane. This increase in pressure Fₚ will push back against the membrane and will decrease the movement thereof relative to the external force Fₑₓₜₑᵣₙₐₗ as the inward deformation amount 240 increases. Thus, if not mitigated, the measurement of the uterine activity by the toco 16 will be inaccurate.

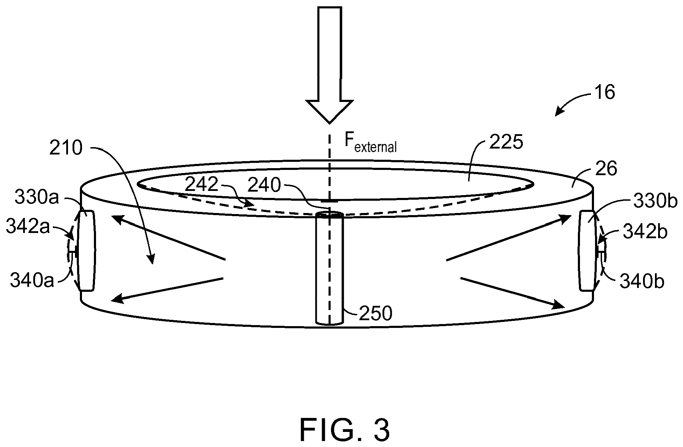

Thus, the disclosed toco 16 is configured to minimize inward deformation of the sensing membrane and/or to compensate for the inward movement of the sensing membrane. In one embodiment, the housing 26 includes one or more pressure diffusing membranes configured to deform outward such that the area inside the sealed housing 26 does not change, or at least does not change sufficiently to impact measurement of the uterine activity by sensors in the sealed cavity. FIG. 3 illustrates such an embodiment, wherein the housing 26 includes two pressure diffusing membranes 330a and 330b configured to expand outward when the sensing membrane deforms inward to mitigate the pressure increase within the sealed cavity 210 due to the inward deformation of the sensing membrane 225. Each pressure diffusing membrane 330a, 330b is configured to expand outward by an amount 340a, 340b that compensates for the amount 240 that the sensing membrane 225 deforms inward in response to an external force Fₑₓₜₑᵣₙₐₗ. The outward expansion of the pressure diffusing membrane 330a and 330b creates excess volumes 342a and 342b that equal the volume 242 displaced by the inward deformation of the sensing membrane 225.

The sensor 250 is located in the sealed cavity of the housing and configured to provide an output based on the inward deformation of the sensing membrane 225. Thus, as the deformation amount of the sensing membrane 225 increases, the output of the sensor 225 also increases. The sensor 250 may be, for example, a spring-loaded linear variable differential transformer (LVDT). In another embodiment, the sensor 250 may be a piezoelectric sensor. Embodiments comprising LVDT sensors and piezoelectric sensors are shown and described herein.

Referring again to FIG. 1, the toco 16 is secured to the abdomen 15 of the maternal patient 14, for example by way of an elastomeric strap 18. However, it will be recognized that in other embodiments, the toco 16 may be secured to the surface of the maternal abdomen 15 by other means, such as by a biocompatible adhesive configured to adhere the toco 16 to the skin of the patient's abdomen 15.

In embodiments, as will be described in further detail herein, the patient monitoring system 10 may be a wireless system wherein the toco 16 and potentially other patient monitoring devices are communicatively connected to the patient monitor 20. As will be understood by the variety of implementations as described in further detail herein, while all remaining within the scope of the present disclosure, the communication link may exemplarily be a wired or a wireless communicative connection. A wireless communication link may be a medical body area network (MBAN), and/or may exemplarily use Bluetooth, Bluetooth Low Energy (BLE), ANT, or ZigBee communication protocols or other RF communication protocols as may be recognized by a person of ordinary skill in the art. In still other exemplary embodiments, depending upon the configuration of the patient monitoring system 10 and the data transmitted between the monitoring system 10 and toco 16, all or some of the data processing of the physiological information acquired by the toco 16 may be performed locally by a processor within the toco 16, such as by a controller located within housing 26 of the toco. The calculated uterine activity parameter(s) may be communicated across the communication link to the external patient monitor 20 exemplarily for visual presentation on a graphical display 24 and/or electronic storage of this information on a data network of the hospital or medical facility and exemplarily in an electronic medical record (EMR) of the maternal patient.

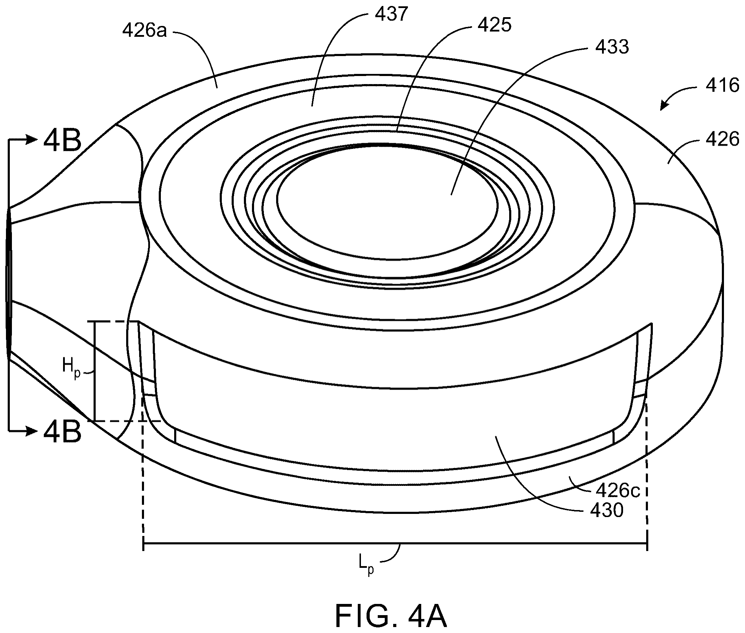

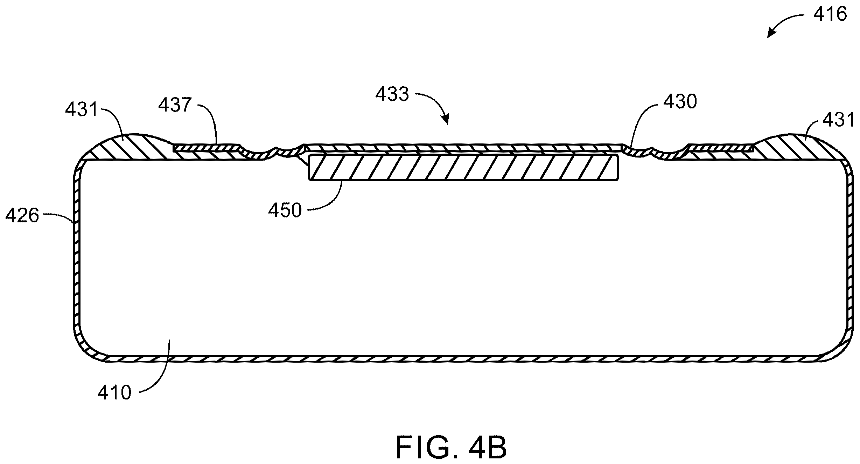

FIGS. 4A-4B depict one embodiment of a toco 416 with a pressure diffusing membrane 430, where FIG. 4A shows a perspective view of the exterior of the toco 416 and FIG. 4B shows a cross sectional view. In the depicted embodiment, the sensing membrane 425 includes a center portion 433 configured to move in response to movement of the maternal abdomen during contractions and includes a stationary portion 437 that is rigid and connects to the rigid housing 426, forming a seal therewith. Specifically, the housing 426 may have a sloped portion 431 configured to connect with and support the outer perimeter of the sensing membrane 425 forming the stationary portion 437.

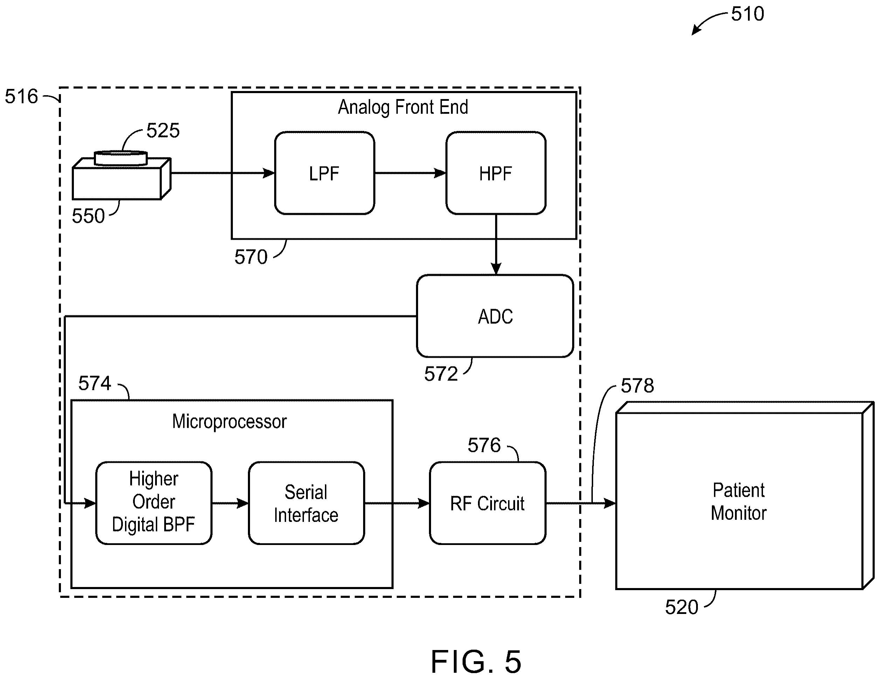

The middle portion 433 has a smaller diameter than the entire sensing membrane 425. In one embodiment, the diameter of the center portion is about 25 mm, whereas the diameter of the sensing membrane is larger than 25 mm. In other embodiments, the movable center portion 433 may be comparatively larger or smaller than the depicted proportions. The sensor 450 is configured to sense movement of the sensing portion 433 and to provide an output proportional thereto. The output is provided to a processor (see FIG. 5), which determines a uterine activity value based thereon.

As discussed above, the inward deformation of the sensing membrane decreases the volume within the cavity 410. The housing 426 of the toco 416 has at least one pressure diffusing membrane 430 to compensate for the decreased volume. The at least one pressure diffusing membrane 430 is constructed of flexible material and is configured to flex outward in response to an inward deflection of the sensing membrane 4425, and particularly the center portion 433, which is the movable portion of the membrane 425.

FIG. 4A shows one embodiment of the pressure diffusing membrane 430 on the lateral side 426c of housing 426. The at least one pressure diffusing membrane 430 is sized to expand to add volume to cavity 410 when the sensing membrane 425 deforms inward to avoid a pressure increase. For example, the materials and size of the pressure diffusing membrane(s) 430 are configured to accommodate the expected volume change of the inward deformation of the sensing membrane 425 as to avoid a pressure increase up to the maximum expected force from a contraction of the maternal patient. The housing 426 includes at least one pressure diffusing membrane 430, and in various embodiments may includes two or more pressure diffusing membranes, such as distributed around the circular lateral side 426c of the housing 426.

The total area of the pressure diffusing membrane(s) 430 is based on the flexibility of the membrane material and the displaced volume of the maximally deformed sensing membrane 425 (which is dependent on the size of the sensing membrane and expected maximum inward deformation thereof). In one embodiment, the material of the pressure diffusing membrane 430 is the same as that used for the sensing membrane 426c. In such an embodiment, the total area of the pressure accommodation membrane(s) 430, which may be the area of one membrane or the areas of a plurality of membranes added together, is greater than or equal to an area of the sensing membrane 425. In the depicted embodiment, the pressure diffusing membrane 430 has a length Lₚ and a height Hₚ. The height Hₚ is less than the height of the housing 426. The length Lₚ may vary depending on the number of pressure diffusing membranes and the amount of volume that needs to be accommodated. Where only one pressure diffusing membrane 430 is provided, the length Lₚ may extend at least halfway around the circumference of the housing 426, such as substantially around the circumference of the housing 426. In another embodiment, the housing 426 may comprise two pressure diffusing membranes 430, one on the first portion of the lateral side 426c, as shown, and a second one on the opposite portion of the lateral side (which is not visible in the figure). In such an embodiment, the length Lₚ of each of the two pressure diffusing membranes 430 is less than half of the circumference of the lateral side 426c, such as each occupying about 25-35% of the circumference. In still other embodiments, three or more pressure diffusing membranes 430 may be distributed around the circular lateral side 426c. The area of the lateral side 426c occupied by the pressure diffusing membranes may be configured to be maximized without compromising the structural integrity of the housing 426 to maintain the shape and volume of the cavity 410. For example, the rigid housing 426 may forms vertical struts between the front side 426a with the sensing membrane 425 and the opposite back side to provide sufficient support to frame openings for the pressure diffusing membranes 430 and maintain the shape and height of the cavity 410.

FIG. 5 depicts one embodiment of a wireless patient monitoring system 510 comprising a wireless toco 516 having a sensing membrane 525 and a sensor 550. The wireless toco 516 includes signal processing elements and electronics within the housing and configured to determine and transmit uterine activity values based on the measurement outputs of the sensor 550. The output of the sensor 550 is received by the analog front end (AFE) 570, which in the depicted example includes a lowpass filter and a high pass filter. The filtered output from the AFE is digitized by the analog-to-digital converter (ADC) 572. The output of the ADC is proved to the processor 574, which is configured to filter the data and determine the uterine activity value(s). Namely, the processor 574 is configured to execute instructions stored on a computer-readable medium to further filter the sensed parameter data and determine the uterine activity accordingly, such as according to known methods for determining uterine activity (UA) values. The wireless toco 516 then transmits the UA value(s) to the patient monitor 520 via RF circuit 576 configured as a transceiver. In other embodiments, the wireless toco 516 may be configured to transmit the digitized and/or filtered sensor output to the patient monitor 520.

The wireless toco 516 and potentially other patient monitoring devices are communicatively connected to the patient monitor 520 by a wireless communication link 578. The wireless communication link 578 may be any of various wireless communication protocols. The wireless communication link 578 may be a medical body area network (MBAN), and/or may exemplarily use Bluetooth, Bluetooth Low Energy (BLE), ANT, or ZigBee communication protocols or other RF communication protocols as may be recognized by a person of ordinary skill in the art. In still other exemplary embodiments, depending upon the configuration of the patient monitoring system. The calculated uterine activity parameter(s) may be communicated across the communication link 578 to the external patient monitor 520 exemplarily for visual presentation on a graphical display and/or electronic storage of this information on a data network of the hospital or medical facility and exemplarily in an electronic medical record (EMR) of the maternal patient.

In various embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

The toco 16, 416, 516 may include any one of various types of sensors configured to sense the movement of the sensing membrane 225, 425, 525 and provide output based thereon. FIGS. 6A and 6B illustrate embodiments of a toco 616a, 616b comprising a spring-loaded LVDT sensor 650 configured to minimize the inward deformation of the sensing membrane 625. Similar to examples described above, the toco 616a, 616b includes a housing 626 that encapsulates a sealed cavity 610, and having a front side 626a that faces the patient, a back side 626b that faces away from the patient, and at least one lateral side 626c connecting therebetween. The front side 626a has a sensing membrane 625. Where the housing 626 is a cylinder shape, the lateral side 626c is one continuous ring-shaped side. Where the housing is a cuboid or generally rectangular, it may comprise multiple sides, wherein some subset of the sides or all of the sides includes a pressure diffusing membrane. Here, there are two pressure diffusing membranes 630a, 630b on the at least one lateral side configured to expand outward with the sensing membrane 625 deforms inward to avoid pressure increase within the sealed cavity 610 that would interfere with the deformation response by the sensing membrane 625.

In the embodiment shown in FIG. 6A, the spring-loaded LVDT 650a includes a core 652a configured to move within a coil 654a. The core 652a is configured to move with the sensing membrane 625, wherein movement of the sensing membrane 625 is translated to the core 652a. Output of the LVDT sensor 650a is based on the movement the core 652a within the coil 654a. A spring 668a is functionally connected to the sensor and configured to absorb and limit the inward deformation of the sensing membrane 625. The spring 668a may be planar spring element having a first portion fixed directly or indirectly to the housing 626 and a second portion fixed to the core 652a (e.g., connected to a flange 653a). Here, the spring 668a is mounted between the PCB 675 and sensing membrane 625. The spring 668a is connected via a plurality of spring supports 669a to the PCB 675. Thus, the spring 668a is indirectly structurally supported by the housing 626 through the PCB supports 676.

In the embodiment shown in FIG. 6B, a different exemplary arrangement of the spring-loaded LVDT sensor 650b is shown. Other arrangements are possible than those shown in FIG. 6A and 6B, and will be evident to a person skilled in the art reviewing the present disclosure. The spring-loaded LVDT 650b includes a core 652b configured to move within a coil 654b. The core 652b is configured to move with the sensing membrane 625, wherein movement of the sensing membrane 625 is translated to the core 652b. Output of the LVDT sensor 650b is based on the movement the core 652b within the coil 654b. A spring 668b is functionally connected to the core 652b and configured to absorb and limit the inward deformation of the sensing membrane 625. The spring 668a may be planar spring element having a first portion fixed directly or indirectly to the housing 626 and a second portion fixed to the core 652b (e.g., connected to a flange 653b). Here, the spring 668b is mounted below the PCB 675, between the battery 680 and the PCB 675. The spring 668b is connected via a plurality of spring supports 669b below the PCB 675. Thus, similar to the design in FIG. 6A, the spring 668b is indirectly structurally supported by the housing 626 through the PCB supports 676.

The spring 668a, 668b is configured to minimize the inward deformation of the sensing membrane 625. The spring 668a, 668b is configured to have a spring constant that decreases movement of the sensing membrane 525 compared to prior art toco systems, thereby minimizing the amount of displaced area that needs to be compensated for by the pressure diffusing membranes 630a, 630b. Whereas existing toco systems implementing spring-loaded LVDT sensors may permit an inward deflection of 2mm using a spring constant of 20lbs/inch, the disclosed toco 616a, 616b is configured to reduce the inward deflection significantly. The inward deflection may be decreased by at least 50% (and thus the displaced volume is likewise decreased), such as to 1 mm of travel in response to maximum expected force from the maternal contractions, by having a spring constant of 50 lbs/inch, which is two times greater than the spring constant of the existing toco. In another embodiment, the inward deflection may be decreased by at least 90% (and thus the displaced volume is likewise decreased), such as to 0.2 mm of travel in response to maximum expected force from the maternal contractions, by having a spring constant of 200 lbs/inch, which is two times greater than the spring constant of the existing toco.

In addition to the spring-loaded LVDT 650a, 650b, the sealed cavity 610 also includes a printed circuit board (PCB) 675 mounted to the housing via a plurality of supports 676, wherein the processor, filtering circuitry, and RF circuitry are mounted. The PCB 675 may also host power management circuitry configured to control the battery 680 and/or distribute power therefrom to the various powered elements within the housing 626.

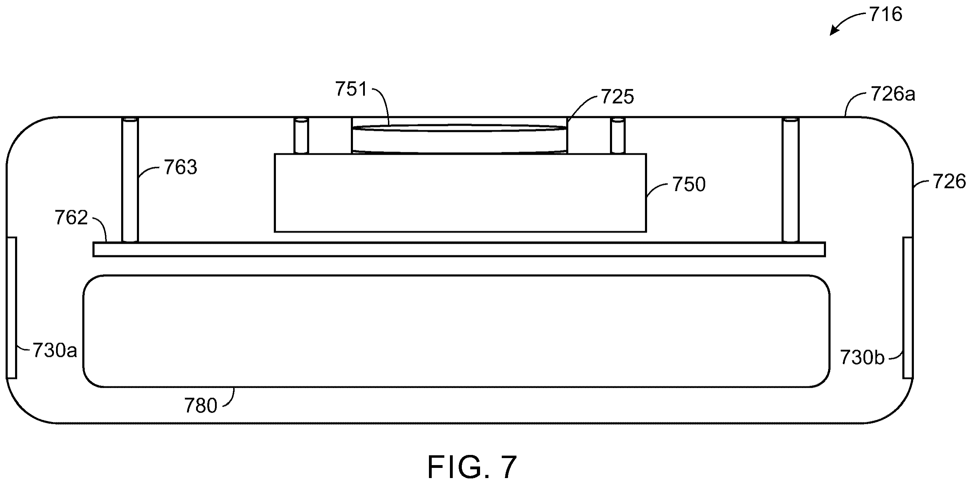

FIG. 7 depicts one embodiment of a toco 716 comprising a temperature compensated piezoelectric sensor 750 configured to minimize the inward deformation of the sensing membrane 725. The temperature compensated piezoelectric sensor 750 is configured to account for the effect of temperature on the output, such as by inclusion of a negative temperature coefficient thermistor or by a passive temperature compensation means to computationally compensate for any shift in output due to temperature change. In one embodiment, the piezoelectric sensor 750 comprises a disc comprised of a deformable plastic outer shell housing an internal cavity filled with a gel material and a piezoelectric material, such as ceramic. When the disc is compressed, the piezo material is deformed and puts out a corresponding voltage.

In the depicted embodiment, a compressible button 751 is between the sensing membrane 725 and the piezoelectric sensor 750. The button 751 is spring loaded such that it distends outward, pushing the center portion of the sensing membrane 725 up above the front side 726a of the housing 726 when not in use on a patient. When the front side 726a is compressed against the patient's abdomen, the disc 751 is depressed into the position shown in FIG. 7, which is then in a position to transmit force between the sensing membrane 725 and the piezoelectric sensor 750.

Since the piezoelectric sensor 750 is highly sensitive to pressure changes, it reacts to compression with almost zero deflection. Thus, the movement of the sensing membrane 725 is minimal and, in some embodiments negligible such that it does not need to be accommodated for by pressure diffusing membrane(s). However, in some embodiments, the pressure diffusing membrane may be included with the piezoelectric sensing embodiment, such as illustrated in FIG. 7 which comprises two pressure diffusing membranes 730a and 730b.

The sealed cavity defined by the housing 726 includes a printed circuit board (PCB) 762 mounted to the housing via a plurality of supports 763. The PCB 762 includes the processor, filtering circuitry, and RF circuitry. The PCB 762 may also host power management circuitry configured to control the battery 780 and/or distribute power therefrom to the various powered elements within the housing 726.

This written description uses examples to disclose the invention(s), including the best mode, and also to enable any person skilled in the art to make and use the invention(s). Certain terms have been used for brevity, clarity, and understanding. No unnecessary limitations are to be inferred therefrom beyond the requirement of the prior art because such terms are used for descriptive purposes only and are intended to be broadly construed. The patentable scope of the invention(s) is defined by the claims and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. A tocodynamometer (toco) configured to measure uterine activity of a maternal patient, the toco comprising:
a housing configured to be maintained against abdominal skin of the maternal patient, wherein the housing forms a sealed cavity and comprises a front side configured to contact the abdominal skin, a back side opposite the front side, and a lateral side connecting the front side to the back side;
a sensing membrane on the front side of the housing, wherein the sensing membrane is configured to be in contact with the abdominal skin and to deform inward during uterine contractions of the maternal patient;
a sensor located in the sealed cavity of the housing and configured to provide an output based on the inward deformation of the sensing membrane;
a processor within the sealed cavity and configured to receive the output of the sensor and to generate a uterine activity value based thereon; and
a pressure diffusing membrane on at least one side of the housing, wherein the pressure diffusing membrane is configured to expand outward when the sensing membrane deforms inward to mitigate pressure increase within the sealed cavity due to the inward deformation of the sensing membrane.

2. The toco of claim 1, wherein the sensor includes a linear variable differential transformer configured to sense the inward deformation of the sensing membrane and a spring functionally connected to the sensor and configured to absorb the inward deformation of the sensing membrane, wherein the spring is configured to minimize the inward deformation of the sensing membrane.

3. The toco of claim 2, wherein the spring is configured to minimize the inward deformation of the sensing membrane due to a maximum expected force from the contractions of the maternal patient to less than 1 mm.

4. The toco of claim 1, wherein the sensor is a piezoelectric sensor which is configured to minimize the inward deformation of the sensing membrane.

5. The toco of claim 1, further comprising at least two pressure accommodation membranes, each on a different portion of the housing.

6. The toco of claim 5, wherein the housing is cylindrical with the front side being flat and the lateral side forming a ring around the front side, wherein the two pressure accommodation membranes are on opposite portions of the lateral side of the housing.

7. The toco of claim 1, wherein the at least one pressure diffusing membrane is configured to expand outward to add a total volume within the sealed cavity that is equal to a volume displaced by the inward deformation of the sensing membrane.

8. The toco of claim 5, wherein a total area of the at least two pressure accommodation membranes is greater than or equal to an area of the sensing membrane.

9. The toco of claim 1, where in the pressure diffusing membrane is comprised of the same material as the sensing membrane.

10. The toco of claim 1, wherein the sealed cavity is watertight.

11. The toco of claim 1, further comprising a battery and a wireless transmitter inside the sealed cavity, wherein the battery is configured to power the processor and the wireless transmitter to wirelessly transmit the uterine activity value to a patient monitor.
